# EUROPÄISCHE PATENTSCHRIFT

(11) **EP 0 622 086 B1**
(45) Veröffentlichungstag und Bekanntmachung des Hinweises auf die Patenterteilung: **05.03.1997**
(21) Anmeldenummer: 94106556.7
(22) Anmeldetag: 27.04.1994
(51) Int. Cl.: A61M 1/16, G01M 3/28

(54) **Hydraulische Sicherheitsschaltung für einen Hämodialyse-Apparat**
Safety hydraulic circuit for an hemodialysis machine
Circuit hydraulique de sécurité pour appareil d'hémodialyse

(30) Priorität: 28.04.1993 DE 4313863; 24.06.1993 DE 4321008
(43) Veröffentlichungstag der Anmeldung: 02.11.1994
(73) Patentinhaber: Fresenius AG, D-61350 Bad Homburg v.d.H (DE)
(72) Erfinder: Baumann, Manfred, D-79422 Schweinfurt (DE); Ender, Helmuth, D-97475 Zeil a.M. (DE)
(74) Vertreter: Fuchs, Luderschmidt & Partner Patentanwälte

(56) Entgegenhaltungen:
- DE-A- 1 927 615
- DE-A- 3 907 490
- DE-C- 3 923 078
- GB-A- 2 045 446

## Beschreibung

Die Erfindung betrifft eine hydraulische Sicherheitsschaltung in Verbindung mit einem Hämodialyse-Apparat mit den weiteren Merkmalen des Oberbegriffs des Anspruchs 1.

Hämodialyse-Apparate haben in den vergangenen Jahrzehnten einen hohen Entwicklungsstand erreicht und werden routinemäßig bei der Behandlung Nierenkranker eingesetzt. Der prinzipielle Aufbau eines Hämodialyse-Apparates ist aus der DE-PS 28 38 414 bekannt.

Während bei Hämodialyse-Apparaten, die als Einzelplatzgeräte ausgelegt sind und im Haus eines nierenkranken Patienten aufgestellt werden, die Dialyseflüssigkeit entweder aus Kanistern angesaugt wird oder aber lediglich ein Dialyseflüssigkeitskonzentrat aus einem Kanister angesaugt und in dem Dialyseapparat mit Wasser vermischt wird, um die zum Betrieb erforderliche Dialyseflüssigkeit herzustellen, hat es sich bei Krankenhäusern mit mehreren Dialyseplätzen als Stand der Technik durchgesetzt, das einzelne Dialysegerät mittels eines Anschlusses mit einer Versorgungsleitung zu verbinden, die als Ringleitung ausgebildet ist. Ein solcher Aufbau einer Mehrplatzanlage zur gleichzeitigen Behandlung mehrerer Patienten ist aus der europäischen Anmeldung EP 0 052 008 bekannt.

Dabei kann die Versorgungsringleitung entweder dazu gedacht sein, den einzelnen Hämodialyse-Apparaten eine fertiggestellte Dialyseflüssigkeit zuzuleiten, oder aber lediglich einzelne Komponenten einer im Dialyseapparat zu mischenden Dialyseflüssigkeit, wie beispielsweise Säure und Bicarbonat. Insbesondere im letzteren Fall kann auch vorgesehen sein, daß mehrere Versorgungs- bzw. Ringleitungen vorgesehen sind, die einzelne Komponenten führen, wobei von jeder Ringleitung zu jedem einzelnen Dialyseapparat eine entsprechende Anzahl von Stichleitungen führt.

Die Versorgungsleitung wird üblicherweise mittels eines Anschlusses, beispielsweise in Form einer Kupplung o. ä. mit dem Dialyseapparat verbunden. Dabei ist in dem Leitungszug von der Versorgungsleitung bis zu den einzelnen Komponenten des Dialyseapparates zumindest ein (erstes) Absperrorgan vorgesehen, um einen Rückfluß von Flüssigkeit in die Versorgungsleitung zu verhindern. Ein solcher Rückfluß ist insbesondere dann zu verhindern, wenn der Hämodialyse-Apparat und das gesamte in ihm enthaltene Rohrleitungssystem mit einer Reinigungs- und/oder Desinfektionslösung durchspült wird, was üblicherweise nach jeder Behandlung durchgeführt wird. Die vergleichsweise aggressive Reinigungs- und Desinfektionsflüssigkeit könnte bei einem Eintreten in die Versorgungsringleitung anderen an die Leitung angeschlossenen Dialyseapparaten zugeführt werden und dort unter Umständen Leben gefährden, wenn an diesen Geräten gerade eine Dialysebehandlung durchgeführt werden würde.

Während ein Rückfluß von Flüssigkeit aus dem Rohrleitungssystem eines Dialyseapparates in die Versorgungsringleitung durch ein einfaches Rückschlagventil verhindert werden könnte, ist es darüber hinaus auch wünschenswert, daß während der Reinigung eines Dialyseapparates keine Dialyseflüssigkeit aus der Versorgungsringleitung in das Rohrleitungssystem des Dialyseapparates fließt, da ein Vermischen von Dialyseflüssigkeit und Reinigungsflüssigkeit zu Reaktionen führen kann, die zu einer Beschädigung des Dialyseapparates führen können.

Um das zuletzt genannte Problem zu lösen, ist es bei Dialyseapparaten gemäß dem Stand der Technik üblich gewesen, anstelle eines einfachen Rückschlagventils ein magnetisch gesteuertes Ventil in den Leitungszug zwischen Versorgungsringleitung und Dialyseapparat einzubauen.

Dabei sperrt das Magnetventil den Leitungszug zwischen Ringleitung und Dialyseapparat in beiden Richtungen und verhindert so ein Fließen von Flüssigkeit. Da aber ein Rückfluß von Desinfektionsflüssigkeit in die Versorgungsleitung Menschenleben gefährden würde und daher auf jeden Fall zu verhindern ist, besteht die Forderung, einen Ausfall des die Leitung sperrenden Magnetventils sofort zu erkennen. Aus Sicherheitsgründen wird daher der Dialyseapparat während der Desinfektion von der Ringleitung getrennt, um eine eventuell auftretende Undichtigkeit des Magnetventils sofort erkennen zu können und einen Rückfluß von Desinfektionsflüssigkeit in die Dialyseflüssigkeit führende Versorgungsringleitung zuverlässig auszuschließen.

Die Trennung des Hämodialyse-Apparates von der Versorgungsringleitung ist jedoch zeitraubend und aufwendig und für das Krankenhauspersonal darüber hinaus lästig, so daß einerseits kostenträchtige Zeitverluste entstehen können, da die nutzbare Betriebszeit des Hämodialyse-Apparates verkürzt wird, andererseits das Krankenhauspersonal verführt sein könnte, auf das Abtrennen des Dialyseapparates von der Versorgungsringleitung im Vertrauen darauf, daß das sperrende Magnetventil ordnungsgemäß arbeitet, zu verzichten, wodurch der angestrebte hohe Sicherheitsstandard gesenkt würde.

Der Erfindung liegt daher die Aufgabe zugrunde, eine Sicherheitsschaltung für einen mittels eines Anschlusses mit einer Versorgungsleitung zu verbindenden Hämodialyse-Apparat zu entwerfen, die einen sehr hohen Sicherheitsstandard ermöglicht und bei der während der Desinfektion ein Abtrennen des Dialyseapparates von der Versorgungsleitung überflüssig ist.

Die Lösung der Aufgabe ist bei einem gattungsgemäßen Hämodialyse-Apparat durch eine hydraulische Sicherheitsschaltung gekennzeichnet, bei der ein zwischen dem Anschluß und dem ersten Absperrorgan angeordnetes zweites Absperrorgan vorgesehen ist, so daß zwischen dem zweiten und dem ersten Absperrorgan ein Puffervolumen gebildet wird, weiter durch Mittel zur Beaufschlagung des Puffervolumens mit einem definierten Druck, weiter durch einen mit dem Puffervolumen verbundenen Druckaufnehmer und durch eine Auswerteeinheit für ein von dem Druckaufnehmer erzeugtes Signal.

Erfindungsgemäß ist dabei bevorzugt vorgesehen, daß das erste Absperrorgan ein Magnetventil ist. Ebenso kann das zweite Absperrorgan ein Magnetventil sein.

Bevorzugt vorgesehen ist, daß das zweite Absperrorgan ein Rückschlagventil ist, und daß der im Puffervolumen eingeschlossene definierte Druck höher ist als der in der Versorgungsleitung herrschende Druck sowie höher als der apparateseitig vor dem Absperrorgan herrschende Spüldruck.

Eine so ausgebildete hydraulische Sicherheitsschaltung gestattet es auf einfache Weise, die ordnungsgemäße Funktion des Absperrorgans zu überwachen. Bei einer Ausgestaltung der erfindungsgemäßen hydraulischen Sicherheitsschaltung, bei der sowohl das erste Absperrorgan wie das zweite Absperrorgan in Form von elektrisch angesteuerten Magnetventilen ausgeführt ist, kann der zwischen diesen beiden Magnetventilen im Puffervolumen eingeschlossene definierte Druck jeden beliebigen Wert annehmen, sofern sich dieser von dem im Hämodialyse-Apparat und in der Versorgungsringleitung herrschenden Druck unterscheidet.

Besonders vorteilhaft ist aber, wenn der für die Beaufschlagung des Puffervolumens gewählte Druck höher ist, als der apparateseitig vor dem Absperrorgan herrschende Spüldruck, sowie höher als der in der Versorgungsleitung herrschende Druck. Bei einer solchen Ausführungsform kann anstelle des zweiten Magnetventils ein Rückschlagventil vorgesehen sein, so daß das Puffervolumen zwischen dem Rückschlagventil und dem ersten Absperrorgan, das weiterhin vorzugsweise in Form eines Magnetventils ausgeführt ist, eingeschlossen ist.

Bei einer solchen Ausführungsform ist ein Abfallen des Druck des Puffervolumens gleichbedeutend mit einem Leck entweder des Absperrorgans in Form des Magnetventils oder aber mit einem Leck des Rückschlagventils. Das von dem Druckaufnehmer erzeugte elektrische Signal wird vorzugsweise mittels eines Mikroprozessors verarbeitet, wodurch ein Absinken des Drucks schnell erkannt werden kann und durch den Mikroprozessor ein Signal ausgegeben werden kann, das die verschiedensten denkbaren Maßnahmen auslösen kann.

Neben der zuletzt geschilderten bevorzugten Ausführungsform ist es weiterhin denkbar, daß der definierte Druck, unter dem das eingeschlossene Puffervolumen steht, - sofern das Puffervolumen zwischen zwei Magnetventilen gebildet wird - entweder niedriger ist als der apparateseitig vor dem Absperrorgan herrschende Spüldruck, oder niedriger als der in der Versorgungsleitung herrschende Druck oder aber niedriger als beide Drücke. Bei einer solchen Ausführungsform wird ein Abweichen des Drucks von dem definierten Druckwert als Fehler interpretiert und durch beispielsweise einen Mikroprozessor die geeigneten Maßnahmen ausgelöst. Bei einer solchen Ausführungsform kann aber in seltenen Fällen, beispielsweise wenn sowohl das erste wie das zweite Absperrorgan ein Leck mit dem gleichen Strömungswiderstand aufweisen, eine Fehlersituation eintreten, die nicht erkannt werden kann. Dies wird durch die erfindungsgemäß bevorzugte Ausführungsform, bei der der in dem Puffervolumen eingeschlossene Druck sowohl höher ist, als der in der Versorgungsleitung herrschende Druck, wie auch höher als der apparateseitig vor dem ersten Absperrorgan herrschende Spüldruck, vermieden.

Auf diese Weise wird eine sichere Möglichkeit geschaffen, ein Versagen des Absperrorgans in kurzer Zeit zu erkennen und entsprechend zu reagieren, weswegen der Dialyseapparat während des Desinfektions- und Reinigungsdurchgangs nicht von der Versorgungsringleitung getrennt werden muß.

Als konkrete Ausgestaltung des Druckaufnehmers sind verschiedene Varianten denkbar. Bei einer Ausführungsform besteht der Druckaufnehmer aus einem druckabhängigen Schalter, der zwischen zwei verschiedenen Schaltzuständen durch Druck hin- und herschaltbar ist und so das Absinken des Druckes unter einem bestimmten Grenzdruck registrieren kann. Der Vorteil einer solchen Ausführungsform ist in erster Linie in geringen Herstellungskosten zu sehen.

Zwar können die Mittel für die Druckbeaufschlagung des zwischen dem Rückschlagventil und dem Absperrorgan angeordneten Puffervolumens beliebig ausgebildet sein, bevorzugt wird jedoch eine Pumpe verwendet. Weiterhin ist bevorzugt vorgesehen, daß eine der in einem Dialyseapparat gemäß dem Stand der Technik bereits vorhandenen Pumpen als Pumpe zur Erzeugung des Drucks verwendet wird. Eine solche Pumpe kann beispielsweise eine Membranpumpe sein, die zur Förderung der Dialyseflüssigkeit vorgesehen ist, oder aber vorzugsweise eine als Zahnradpumpe ausgebildete Entgasungspumpe, die die für die Behandlung notwendige Dialyseflüssigkeit durch eine Entgasungsstrecke treibt.

Vorzugsweise ist dabei die Druckseite der Pumpe über ein Magnetventil direkt oder indirekt mit dem apparateseitigen Anschluß des das Puffervolumen einschließenden Absperrorgans, d.h. des ersten Magnetventils verbindbar. Das erstgenannte Magnetventil wird im folgenden auch als "drittes" Magnetventil bezeichnet werden, als Abgrenzung gegen das an der Schnittstelle zwischen Versorgungsleitung und Dialyseapparat angeordnete "erste" Magnetventil, sowie das gegebenenfalls anstelle des erwähnten Rückschlagventils vorhandene "zweite" Magnetventil.

Durch eine solche Anordnung kann der von der Entgasungspumpe aufgebaute Druck dazu verwendet werden, das Puffervolumen mit Druck zu beaufschlagen, wodurch die Notwendigkeit zur Installation einer zweiten Pumpe entfällt.

Eine zusätzliche Pumpe oder eine andere Druckerzeugungsvorrichtung entfällt somit.

Erfindungsgemäß kann weiterhin vorgesehen sein, daß ein Druckbegrenzungsventil direkt oder indirekt mit dem apparateseitigen Anschluß des das Puffervolumen begrenzenden ersten Absperrorgans, d.h. dem ersten Magnetventil, verbunden ist.

Weiterhin kann ein im folgenden als "viertes" bezeichnetes Magnetventil vorgesehen sein, durch das die Saugseite der Entgasungspumpe direkt oder indirekt mit dem apparateseitigen Anschluß des das Puffervolumen abschließenden ersten Absperrorgans, d.h. des ersten Magnetventils verbindbar bzw. von diesem trennbar ist. Der Vorteil einer solchen Anordnung liegt darin, daß bei einer Betriebsweise der Sicherheitsschaltung bzw. des gesamten Dialyseapparates, bei dem zur Bildung des Puffervolumens Osmoseflüssigkeit in den ersten Abschnitt der Versorgungsleitung bis zu dem Rückschlagventil oder bis zu dem gegebenenfalls stattdessen vorgesehenen "zweiten" Magnetventil gedrückt wird, diese wieder abgesaugt werden kann, so daß sie sich bei einem nachfolgenden Dialysevorgang nicht mit Dialyseflüssigkeit vermischt.

Wie bereits erwähnt, ist vorteilhafterweise vorgesehen, daß die Sicherheitsschaltung einen Mikroprozessor aufweist, der das von dem Druckabnehmer generierte Signal überwacht und weiterverarbeitet. Der Mikroprozessor kann vorteilhafterweise mit dem Motor der Pumpe bzw. mit einer Motorsteuerung verbunden sein, so daß im Störfalle der Motor umgehend abgeschaltet wird, wodurch eine weitere Förderung von Desinfektionsflüssigkeit unterbunden wird.

Erfindungsgemäß ist weiterhin vorgesehen, daß der Mikroprozessor elektrisch mit den drei genannten (erstes, drittes, viertes) Magnetventilen verbunden ist und daß Befehle zum Öffnen und Schließen der Magnetventile erzeugt und verarbeitet werden können. Durch diese Ausführungsform der Erfindung läßt sich der gesamte zum Betrieb der Sicherheitsschaltung notwendige Verfahrensablauf einschließlich des Aufbauens des Drucks des Puffervolumens durch ein entsprechendes im Mikroprozessor abgelegtes Programm automatisieren.

Beispielsweise kann insbesondere bei der zuletztgenannten Variante, bei der der Mikroprozessor mit der Motorsteuerung der zum Aufbauen des Drucks verwendeten Pumpe verbunden ist, vorgesehen sein, daß die zu Beginn des Desinfektionsvorgangs, d.h. beim Aufbauen des Drucks des Puffervolumens, geförderte Menge überwacht wird, beispielsweise indem die Umdrehungen der Pumpenwelle gezählt werden. Auf diese Weise kann sichergestellt werden, daß auch in dem Fall, daß das zweite Absperrorgan bzw. Rückschlagventil defekt ist, nur eine begrenzte Menge von Osmoseflüssigkeit in das Puffervolumen gedrückt wird.

Alternativ kann der Mikroprozessor auch dazu verwendet werden, die Phase des Druckaufbaus im Puffervolumen zu Beginn des Spülprogramms zu überwachen. Sollte der unwahrscheinliche Fall eintreten, daß bei geöffnetem ersten Absperrventil das Rückschlagventil defekt ist, so kommt es zu keinem Druckaufbau im Puffervolumen, da die geförderte Osmoseflüssigkeit in die Versorgungsringleitung gedrückt wird. Der Druckschalter oder Drucksensor gibt dementsprechend kein einem Druckaufbau entsprechendes Signal an den Mikroprozessor. Während das Versagen des Rückschlagventils eine der Möglichkeiten ist, warum der Drucksensor kein Drucksignal an den Mikroprozessor liefert, kann eine zweite Möglichkeit sein, daß der Drucksensor selbst defekt ist. Es kann daher erfindungsgemäß vorgesehen sein, daß in der Anfangsphase des Spülprogramms, d.h. wenn ein Druck im Puffervolumen aufgebaut werden soll, ein Ausbleiben des Signal des Drucksensors zunächst als Indiz für einen defekten Drucksensor gewertet wird. Auf eine entsprechende Anzeige hin, die vom Mikroprozessor veranlaßt wird, kann dann in einem ersten Schritt zunächst der Drucksensor ausgetauscht werden. Bleibt bei einem neuerlichen Versuch, einen Druck im Puffervolumen während der Anfangsphase des Spülprogramms aufzubauen, das entsprechende Drucksignal vom Drucksensor weiterhin aus, so ist damit zu rechnen, daß das Rückschlagventil, oder bei einer entsprechenden Ausführungsform der Erfindung, das zweite Magnetventil, d.h. das zweite Absperrorgan, defekt ist.

In jedem Fall wird von dem Mikroprozessor das erste Absperrorgan geschlossen und das Spülprogramm unterbrochen, wenn während des Aufbaus des Drucks im Puffervolumen ein entsprechendes Drucksignal des Drucksensors ausbleibt.

Die erfindungsgemäße Sicherheitsschaltung läßt sich darüber hinaus auch als Druckminderer betreiben, falls der in der Versorgungsleitung herrschende Versorgungsdruck P_{V} höher ist, als ein definierter Grenzwert. Hierzu kann das mit dem Mikroprozessor verbundende erste Absperrorgan (erstes Magnetventil) intermittierend angesteuert werden, so daß der im Puffervolumen sich einstellende Druck gleich oder kleiner ist als der definierte Grenzwert. Die Anpassung des in der Versorgungsleitung für Dialyseflüssigkeit herrschenden Versorgungsdrucks P_{V} an einen wünschenswerten kleineren Druck kann auch dazu verwendet werden, Fördercharakteristiken der in dem Dialyseapparat angeordneten Membranpumpe zu beeinflussen.

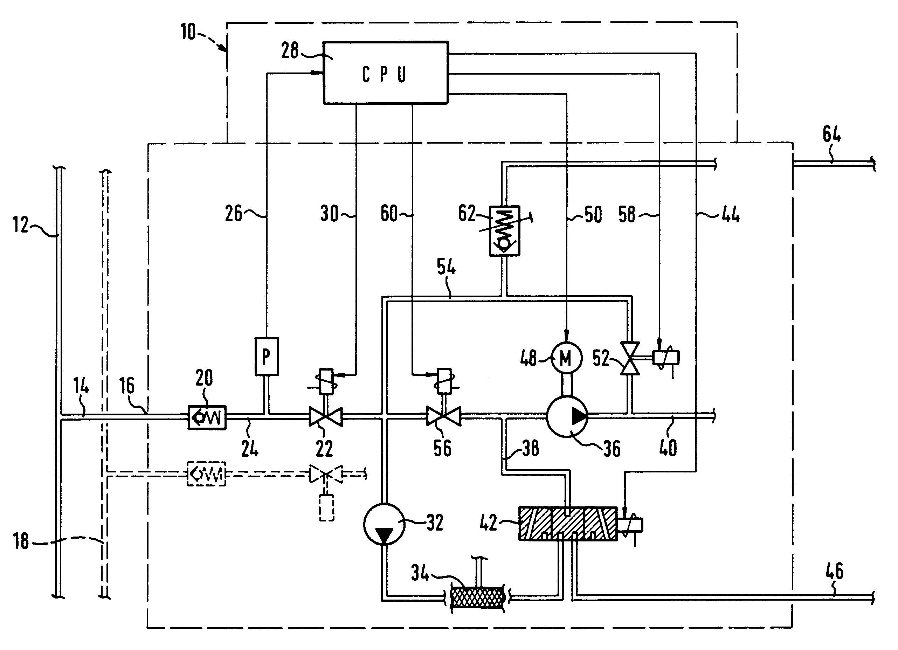

Das bevorzugte Verfahren zum Spülen des Rohrleitungssystems eines Hämodialyse-Apparates gemäß der Erfindung mit einer Reinigungs- und/oder Desinfektionsflüssigkeit bei gleichzeitiger Überwachung der ordnungsgemäßen Funktion des Absperrorgans (erstes Magnetventil) der Versorgungsleitung wird weiter unten stehend anhand der Zeichnung näher beschrieben.

In der Zeichnung zeigt die einzige Figur eine schematische Darstellung der für die Erfindung wesentlichen hydraulischen und elektrischen Schaltungskomponenten eines erfindungsgemäßen Hämodialyse-Apparates, wobei nicht dargestellte Komponenten, die für die eigentliche Dialyse notwendig sind, wie beispielsweise Membranen, Bilanzierkammern etc. durch ein schraffiertes Feld lediglich angedeutet sind, um die Klarheit der Zeichnung zu bewahren.

Ein erfindungsgemäßes Dialysegerät 10 ist an eine Versorgungsringleitung 12 angeschlossen, von der eine Stichleitung 14 abgeht und an einer Anschlußstelle 16 mit dem Dialyseapparat 10 verbunden ist. Wie durch eine zweite gestrichelte Versorgungsringleitung 18 angedeutet, kann der Dialyseapparat 10 an mehrere Versorgungsringleitungen in der erwähnten Art und Weise angeschlossen sein, wobei beispielsweise eine erste Versorgungsringleitung 12 eine saure Lösung und eine zweite Versorgungsringleitung 18 Bicarbonat-Lösung führt. Stromabwärts der Stichleitung 14 schließt sich hinter der Anschlußstelle 16 apparateseitig ein Rückschlagventil 20 an, das von einem ersten Magnetventil 22 gefolgt wird. Wie bereits ausgeführt, kann jedoch anstelle des Rückschlagventils 20 auch ein zweites Magnetventil vorgesehen sein. Zwischen dem Rückschlagventil 20 und dem ersten Magnetventil 22 ist ein Puffervolumen 24 eingeschlossen, das in Form eines Rohrleitungsabschnitts oder eines widerstandsfähigen Schlauchs ausgeführt ist und ein Volumen von wenigen Millilitern umfaßt. An das Puffervolumen 24 angeschlossen ist ein Drucksensor P, der den im Puffervolumen herrschenden Druck aufnimmt und in ein elektrisches Signal umsetzt, das über eine Leitung 26 einem Mikroprozessor 28 zugeführt wird. Das erste Magnetventil 22 ist ebenfalls über eine Leitung 30 mit dem Mikroprozessor verbunden. Sollte anstelle des Rückschlagventils 20 ein zweites Magnetventil vorgesehen sein, so ist dies ebenfalls über eine Leitung mit dem Mikroprozessor verbunden. Hinter dem ersten Magnetventil 22 ist eine Membranförderpumpe 32 angeschlossen, die die in der Versorgungsringleitung 12 geförderte Flüssigkeit, sei es Dialyseflüssigkeit oder ein Dialyseflüssigkeitsbestandteil, in das nicht näher dargestellte Rohrleitungssystem 34 pumpt, das insgesamt aus dem Stand der Technik bekannt ist.

Eine zweite Pumpe 36 dient als sogenannte Entgasungspumpe und saugt über eine Leitung 38 aus dem nicht näher erläuterten Rohrleitungssystem 34 des Dialyseapparates Dialyseflüssigkeit an und treibt diese über eine Rohrleitung 40 zu einer nicht näher dargestellten Entgasungsstrecke. Bei der beispielhaft dargestellten Ausführungsform eines erfindungsgemäßen Hämodialyse-Apparates wird hierzu ein 3/2-Wegeventil 42 in die entsprechende Stellung geschaltet. Das 3/2-Wegeventil ist über eine Leitung 44 ebenfalls mit dem Mikroprozessor 28 (CPU) verbunden.

In einer anderen Stellung verbindet das 3-Wegeventil 42 die Ansaugleitung 38 der Entgasungspumpe 36 mit einer Zufuhrleitung 46 für Reinigungs- und/oder Desinfektionsflüssigkeit.

Ein Motor 48 der Pumpe 36 ist über eine Leitung 50 mit dem Mikroprozessor 28 verbunden.

Über ein drittes Magnetventil 52 und eine Leitung 54 ist die Druckseite der Entgasungspumpe 36 mit dem apparateseitigen Anschluß des ersten Magnetventils 22 verbunden.

Bei diesem Ausführungsbeispiel mit dem ersten Magnetventil 22 in Serie geschaltet ist ein viertes Magnetventil 56, das zur Saugseite der Entgasungspumpe 36 führt. Erstes, drittes und viertes Magnetventil 22, 52 und 56 sind über elektrische Leitungen 30, 58 und 60 mit dem Mikroprozessor verbunden.

An die von der Druckseite der Entgasungspumpe 36 über das dritte Magnetventil 52 zum ersten Magnetventil 22 führende Leitung 54 ist ein Überdruckventil 62 angeschlossen, das beispielsweise bei einem Druck von etwa 1,2 bar öffnet.

Symbolisch angedeutet ist, daß der gesamte Dialyseapparat 10 über einen Abfluß 64 verfügt. Ebenso wie durch das symbolhaft dargestellte Rohrleitungssystem 34 soll dadurch angedeutet werden, daß der gesamte hydraulische Schaltungsaufbau des erfindungsgemäßen Hämodialyse-Apparates wesentlich komplexer ist, als hier dargestellt.

Während des normalen Betriebes, d.h. während der Dialyse, ist das erste Magnetventil 22 durchgeschaltet, das vierte Magnetventil 56 ist gesperrt, das 3-Wege-Ventil 42 ist so geschaltet, daß die Ansaugleitung 38 der Entgasungspumpe 36 mit dem dafür vorgesehenen, hier nicht näher dargestellten Teil des Rohrleitungssystems 34 in Verbindung steht und das dritte Magnetventil 52 ist ebenfalls gesperrt.

Die Dialyseflüssigkeit bzw. eine ihrer Komponenten gelangt aus der Versorgungsringleitung 12 über die Stichleitung 14 und den Anschluß 16 durch das Rückschlagventil 20 in den das Puffervolumen bildenden Rohrleitungsteil 24, von dort aus über das geöffnete erste Magnetventil 22 zur Ansaugseite der Membranförderpumpe 32. Die Membranförderpumpe 32 drückt die Dialyseflüssigkeit in das Rohrleitungssystem 34, 38, wo der hier nicht näher beschriebene Dialyseprozeß gemäß dem Stand der Technik ausgeführt wird. Nach erfolgter Dialyse verläßt das belastete Dialysat den Dialyseapparat über die Entsorgungsleitung 64.

Soll der Dialyseapparat nach erfolgter Dialyse desinfiziert und gereinigt werden, so wird auf entsprechende Veranlassung des Mikroprozessors (CPU) 28 hin folgendes Verfahren automatisch ausgeführt:

Zunächst ist das vierte Magnetventil 56 wie gehabt geschlossen und das 3-Wegeventil 42 ist so geschaltet, daß die Ansaugseite der Entgasungspumpe 36 über die Leitung 38 mit dem Rohrleitungssystem 34 verbunden ist. Das dritte Magnetventil 52 wird nun geöffnet, und Osmoseflüssigkeit aus dem Rohrleitungssystems 34 angesaugt und über die Leitung 54 durch das erste Magnetventil 22 in das Puffervolumen 24 bis zum Rückschlagventil 20 gedrückt.

Während dieser Phase des Druckaufbaus im Puffervolumen 24 wird das vom Drucksensor P gelieferte Signal 26 durch den Mikroprozessor 28 überwacht. Bleibt ein Druckanstieg aus, so wird zunächst vermutet, daß der Drucksensor P defekt ist und das Spülprogramm unterbrochen. Eine entsprechende Fehlermeldung wird ausgegeben, die darauf hinweist, daß der Drucksensor auszutauschen ist.

Nach Austauschen des Drucksensors wird der Vorgang des Druckaufbaus mittels von Entgasungspumpe 36 geförderter Osmoseflüssigkeit wiederholt. Kommt es wiederum zu einem Ausbleiben eines den Druckaufbau signalisierenden Signals vom Drucksensor P, so wird diesmal vermutet, daß das Rückschlagventil 20 defekt ist und ebenfalls das Spülprogramm unterbrochen.

In dem Fall, daß ein ordnungsgemäßer Druckanstieg vorliegt, fördert die Entgasungspumpe 36 solange Osmoseflüssigkeit über das geöffnete dritte Magnetventil 52, bis das Druckbegrenzungsventil 62 öffnet und weiterhin geförderte Osmoseflüssigkeit über die sich daran anschließende Leitung abfließt. Der Drucksensor P meldet nun das Erreichen des definierten Drucks an den Mikroprozessor 28, woraufhin das erste Absperrorgan, d.h. das erste Magnetventil 22 geschlossen wird. Im Puffervolumen 24 herrscht nun ein definierter Druck, der höher ist, als der in der Versorgungsleitung 12 bzw. Stichleitung 14 herrschende Versorgungsdruck P_{V}, sowie höher ist, als der später zum Spülen des Systems verwendete Spüldruck P_{S}. Kommt es im nun folgenden Desinfektionsvorgang zu einem Abfallen dieses Drucks P_{PV}, so kann davon ausgegangen werden, daß Flüssigkeit entweder über das erste Magnetventil oder über das Rückschlagventil 20 abfließt, gleichbedeutend mit einem Leck bzw. mit einem Fehler.

Zur Durchführung des eigentlichen Spülvorgangs wird das 3-Wegeventil 42 getaktet. Zunächst wird es so geschaltet, daß die Ansaugseite der Entgasungspumpe 36 über die Rohrleitung 38 und das 3-Wegeventil 42 mit der Desinfektionsflüssigkeits-Ansaugleitung 46 verbunden ist. Es wird Desinfektionsflüssigkeit angesaugt und bei geschlossenem ersten Absperrorgan, d.h. erstem Magnetventil über die Leitung 54 und die Membranpumpe 32 in das zu desinfizierende Rohrleitungssystem 34 gefördert. Das 3-Wegeventil 42 schließt nun wieder, so daß die Ansaugseite der Entgasungspumpe 36 über die Leitung 38 mit dem Rohrleitungssystem 34 in Verbindung steht. Die Membranpumpe 32 fördert die Desinfektionsflüssigkeit durch das zu desinfizierende Rohrleitungssystem 34. Bei Bedarf kann der Vorgang wiederholt werden.

Bei der beispielhaft dargestellten Ausführungsform eines erfindungsgemäßen Hämodialyse-Apparates ist es weiterhin möglich, das dritte Magnetventil und das vierte Magnetventil gegenläufig abwechselnd zu schließen und zu öffnen. Durch dieses Takten der beiden genannten Magnetventile wird sichergestellt, daß die umgepumpte Desinfektionsflüssigkeit sowohl das Rohrleitungssystem 34 wie auch die Leitung 54 durchspült, so daß der gesamte Hämodialyse-Apparat desinfiziert wird.

Nachdem der Spülvorgang abgeschlossen ist, kann von dem Mikroprozessor 28 ein Signal generiert werden, daß das vierte Magnetventil 56 öffnet, ebenso wie das erste Absperrorgan bzw. das erste Magnetventil 22. Bei geschlossenem dritten Magnetventil 52 kann dann die im Puffervolumen 24 enthaltene Flüssigkeit abgesaugt werden, im vorliegenden Fall mittels der Entgasungspumpe 36, bevor der Hämodialyse-Apparat wieder auf den Standardbetrieb umgeschaltet wird, bei dem Dialyseflüssigkeit aus der Ringleitung 12 über die Stichleitung 14, das Rückschlagventil 20, das Puffervolumen 24 und das erste Magnetventil 22 gefördert wird und durch das Rohrleitungssystem 34 fließt.

Während des gesamten Spülvorgangs wird das von dem Drucksensor P generierte Signal überwacht und somit sichergestellt, daß das erste Absperrorgan in Form des ersten Magnetventils 22 ordnungsgemäß arbeitet und eine strikte Trennung zwischen Versorgungsleitung 12 und Hämodialyse-Apparat gewährleistet ist, solange sich in diesem Desinfektionsflüssigkeit befindet. Die Sicherheit der Anlage wird auf diese Weise deutlich gesteigert, ohne daß es notwendig wäre, den Hämodialyse-Apparat vollständig vom Versorgungsnetz zu trennen, um den Desinfektionsvorgang durchzuführen.

### Bezugszeichenliste

- 10: Dialyseapparat
- 12: Versorgungsringleitung
- 14: Stichleitung
- 16: Anschlußstelle
- 20: zweites Absperrorgan (Rückschlagventil oder zweites Magnetventil)
- 22: erstes Absperrorgan (erstes Magnetventil)
- 24: Puffervolumen
- 26: Leitung (von P)
- 28: Mikroprozessor (CPU)
- 30: Leitung (zwischen CPU und 22)
- 32: Membranförderpumpe
- 34: Rohrleitungssystem
- 36: Entgasungspumpe
- 38: Ansaugleitung (von 36)
- 40: Entgasungsstrecke
- 42: 3/2-Wegeventil
- 44: Leitung (zwischen CPU und 42)
- 46: Zufuhrleitung für Desinfektionsflüssigkeit
- 48: Motor (von 36)
- 50: Leitung (zwischen CPU und 48)
- 52: drittes Magnetventil
- 54: Druckleitung
- 56: viertes Magnetventil
- 58: Leitung (zwischen CPU und 52)
- 60: Leitung (zwischen CPU und 56)
- 62: Rückschlagventil
- 64: Abflußleitung

## Patentansprüche

1. Hydraulische Sicherheitsschaltung in Verbindung mit einem Hämodialyse-Apparat (10), der mittels eines ersten Anschlusses (16) mit einer Versorgungsleitung (12) zu verbinden ist, der apparateseitig vor dem Anschluß (16) ein erstes Absperrorgan (22) sowie ein sich daran anschließendes verzweigtes Rohrleitungssystem (34) aufweist, das über einen zweiten Anschluß (46) mit einer Reinigungs- und/oder Desinfektionsflüssigkeit durchspült werden kann,
gekennzeichnet durch
ein zwischen dem ersten Anschluß (16) und dem ersten Absperrorgan (22) angeordnetes zweites Absperrorgan (20), das in Richtung von dem Hämodialyse-Apparat (10) zur Versorgungsleitung (12) sperrt, so daß ein zwischen dem zweiten Absperrorgan (20) und dem ersten Absperrorgan (22) angeordnetes Puffervolumen (24) gebildet wird,
Mittel (36) zur Beaufschlagung des Puffervolumens (24) mit einem definierten Druck,
einen mit dem Puffervolumen (24) verbundenen Druckaufnehmer (P),
und eine Auswerteeinheit für ein von dem Druckaufnehmer (P) erzeugtes Signal.

2. Hydraulische Sicherheitsschaltung nach Anspruch 1, dadurch gekennzeichnet, daß das erste Absperrorgan (22) ein Magnetventil (erstes Magnetventil) ist.

3. Hydraulische Sicherheitsschaltung nach einem der Ansprüche 1 oder 2, dadurch gekennzeichnet, daß das zweite Absperrorgan (20) ein Magnetventil (zweites Magnetventil) ist.

4. Hydraulische Sicherheitsschaltung nach einem der Ansprüche 1 oder 2, dadurch gekennzeichnet, daß das zweite Absperrorgan (20) ein Rückschlagventil ist und daß der in dem Puffervolumen (24) erzeugte definierte Druck höher ist als der in der Versorgungsleitung (12) herrschende Druck sowie höher als der apparateseitig vor dem ersten Absperrorgan (22) herrschende Spüldruck.

5. Sicherheitsschaltung nach einem der vorhergehenden Ansprüche, dadurch gekennzeichnet, daß der Druckaufnehmer (P) ein analoger Drucksensor ist.

6. Sicherheitsschaltung nach einem der Ansprüche 1 bis 4, dadurch gekennzeichnet, daß der Druckaufnehmer (P) ein druckabhängiger Schalter ist.

7. Sicherheitsschaltung nach einem der vorhergehenden Ansprüche, dadurch gekennzeichnet, daß das Mittel (36) zur Druckbeaufschlagung des Puffervolumens (24) eine Pumpe (36) ist.

8. Sicherheitsschaltung nach Anspruch 7, dadurch gekennzeichnet, daß die Pumpe (36) eine Membranpumpe des Dialyseapparates ist.

9. Sicherheitsschaltung nach Anspruch 7, dadurch gekennzeichnet, daß eine in dem Dialyseapparat (10) angeordnete Entgasungspumpe (36) zum Treiben der Dialyseflüssigkeit durch eine Entgasungsstrecke (40) als Pumpe (36) zur Druckbeaufschlagung des Puffervolumens (24) schaltbar ist.

10. Sicherheitsschaltung nach einem der Ansprüche 7 bis 9, dadurch gekennzeichnet, daß die Druckseite der Pumpe (36) über ein drittes Magnetventil (52) direkt oder indirekt mit dem apparateseitigen Anschluß des das Puffervolumen (24) einschließenden ersten Abspertorgans (22) verbindbar ist.

11. Sicherheitsschaltung nach Anspruch 10, gekennzeichnet durch ein mit dem apparateseitigen Anschluß des das Puffervolumen (24) begrenzenden ersten Absperrorgans (22) verbundenes Druckbegrenzungsventil (62).

12. Sicherheitsschaltung nach Anspruch 11, dadurch gekennzeichnet, daß der Auslösewert des Druckbegrenzungsventils (62) 1,2 bar beträgt.

13. Sicherheitsschaltung nach einem der Ansprüche 7 bis 9, dadurch gekennzeichnet, daß die Saugseite der Pumpe (36) direkt oder indirekt über ein viertes Magnetventil (56) mit dem apparateseitigen Anschluß des das Puffervolumen (24) abschließenden ersten Absperrorgans (22) verbindbar ist.

14. Sicherheitsschaltung nach einem der Ansprüche 7 bis 9, dadurch gekennzeichnet, daß ein Mikroprozessor (28) mit dem Motor der Pumpe (36) verbunden ist.

15. Sicherheitsschaltung nach Anspruch 14, dadurch gekennzeichnet, daß der Mikroprozessor (28) mit dem dritten Magnetventil (52) elektrisch verbunden ist.

16. Sicherheitsschaltung nach Anspruch 14, dadurch gekennzeichnet, daß der Mikroprozessor (28) mit dem vierten Magnetventil (56) elektrisch verbunden ist.

17. Hydraulische Sicherheitsschaltung nach Anspruch 14, dadurch gekennzeichnet, daß der Mikroprozessor (28) mit dem ersten Magnetventil (22) verbunden ist und die Kombination aus Druckaufnehmer (P), erstem Magnetventil (22) und Mikroprozessor (28) als Druckminderer arbeitet, wenn der Hämodialyse-Apparat (10) im regulären Dialyseverfahren betrieben wird und der Druck P_{V} in der Versorgungsleitung (12) der Dialyseflüssigkeit höher liegt als ein vorgegebener Grenzwert.

18. Verfahren zum Spülen des Rohrleitungssystems (34) eines Hämodialyse-Apparates (10) mit Sicherheitsschaltung nach einem oder mehreren der Ansprüche 10 bis 17 mit einer Reinigungs- und/oder Desinfektionsflüssigkeit bei gleichzeitiger Überwachung der ordnungsgemäßen Funktion des ersten Absperrorgans (22) der Versorgungsleitung (12), gekennzeichnet durch folgende Verfahrensschritte:
- Öffnen des ersten Magnetventils (22),
- Öffnen des dritten Magnetventils (52),
- Ansaugen von Osmoseflüssigkeit mittels der Entgasungspumpe (36) aus dem Rohrleitungssystem (34) des Hämodialyse-Apparates (10),
- Füllen des Puffervolumens (24) und Aufbauen eines Vordrucks, bis das Druckregelventil öffnet,
- Schließen des ersten Magnetventils (22),
- Umschalten der Ansaugseite der Entgasungspumpe (36) von dem Rohrleitungssystem (34) auf eine Zuführleitung (46) für Desinfektionsflüssigkeit,
- Ansaugen von Desinfektionsflüssigkeit,
- Umschalten der Ansaugseite der Entgasungspumpe (36) von der Zufuhrleitung (46) für Desinfektionsflüssigkeit auf das Rohrleitungssystem (34),
- Umwälzen der Desinfektionsflüssigkeit und Spülen des Rohrleitungssystems (34) mit einem Spüldruck P_{S} < P_{PV},
- Überwachung des von dem Druckaufnehmer (P) gelieferten Signals und Abschalten der die Desinfektionsflüssigkeit treibenden Pumpe (36), sobald der Druck des Puffervolumens (24) unter einen vorgegebenen Grenzwert fällt,
- Öffnen des ersten Magnetventils (22),
- Absaugen des Puffervolumens (24),
- und Ansaugen von Dialyseflüssigkeit aus der Versorgungsleitung (12).

## Claims

1. Hydraulic safety circuit in conjunction with a haemodialysis apparatus (10) which is to be connected by means of a first connection (16) with a supply pipeline (12) which has on the apparatus side in front of the connection (16) a first shut-off device (22) as well as a branched piping system (34) connected to this which can be thoroughly rinsed by way of a second connection (46) with a cleansing and/or disinfecting fluid,
characterized by
a second shut-off device (20) located between the first connection (16) and the first shut-off device (22), this second shut-off device (20) closing in direction from the haemodialysis apparatus (10) to the supply pipeline (12), so that a buffer volume (24) is formed between the second shut-off device (20) and the first shut-off device (22),
means (36) For pressurizing the buffer volume (24) to a specific pressure,
a pressure sensor (P) connected with the buffer volume
and an evaluating unit for a signal produced by the pressure sensor (P).

2. Hydraulic safety circuit according to Claim 1, characterized in that the first shut-off device (22) is a magnetic valve (first magnetic valve).

3. Hydraulic safety circuit according to one of Claims 1 or 2, characterized in that the second shut-off device (20) is a magnetic valve (second magnetic valve).

4. Hydraulic safety circuit according to one of Claims 1 or 2, characterized in that the second shut-off device (20) is a non-return valve and that the specific pressure produced in the buffer volume (24) is higher than the pressure prevailing in the supply pipeline (12) and higher than the rinsing pressure prevailing on the apparatus side in front of the first shut-off device (22).

5. Safety circuit according to one of the preceding claims, characterized in that the pressure sensor (P) is an analogue pressure sensor.

6. Safety circuit according to one of Claims 1 to 4, characterized in that the pressure sensor (P) is a pressure-dependent switch.

7. Safety circuit according to one of the preceding claims, characterized in that the means (36) for pressurizing the buffer volume (24) is a pump (36).

8. Safety circuit according to Claim 7, characterized in that the pump (36) is a diaphragm pump of the dialysis apparatus.

9. Safety circuit according to Claim 7, characterized in that a degassing pump (36), located in the dialysis apparatus for driving the dialysis fluid through a degassing section (40), can be switched as pump (36) for pressurizing the buffer volume (24).

10. Safety circuit according to one of Claims 7 to 9, characterized in that the delivery side of the pump (36), by way of a third magnetic valve (52), can be connected directly or indirectly with the connection on the apparatus side of the first shut-off device (22) containing the buffer volume (24).

11. Safety circuit according to Claim 10, characterized by a pressure-limiting valve (62) connected with the connection on the apparatus side of the first shut-off device (22) which limits the buffer volume (24).

12. Safety circuit according to Claim 11, characterized in that the trigger value of the pressure-limiting valve (62) amounts to 1.2 bar.

13. Safety circuit according to one of Claims 7 to 9, characterized in that the suction side of the pump (36) can be directly or indirectly connected by way of a fourth magnetic valve (56) with the connection on the apparatus side of the first shut-off device (22) which closes off the buffer volume (24).

14. Safety circuit according to one of Claims 7 to 9, characterized in that a microprocessor (28) is connected with the motor of the pump (36).

15. Safety circuit according to Claim 14, characterized in that the microprocessor (28) is electrically connected with the third magnetic valve (52).

16. Safety circuit according to Claim 14, characterized in that the microprocessor (28) is electrically connected with the fourth magnetic valve (56).

17. Hydraulic safety circuit according to Claim 14, characterized in that the microprocessor (28) is connected with the first magnetic valve (22) and the combination of pressure sensor (P), first magnetic valve (22) and microprocessor (28) works as a pressure reducer, when the haemodialysis apparatus (10) is being operated for the usual dialysis procedure and the pressure Pᵥ in the supply pipeline (12) of the dialysis fluid is higher than a predetermined limiting value.

18. Method of rinsing the piping system (34) of a haemodialysis apparatus (10), with safety circuit according to one or more of Claims 10 to 17, with a cleansing and/or disinfecting fluid with simultaneous monitoring of the proper functioning of the first shut-off device (22) of the supply pipeline (12), characterized by the following procedural steps:
- Opening of the first magnetic valve (22),
- Opening of the third magnetic valve (52),
- Drawing off of osmosis fluid by means of the degassing pump (36) from the piping system (34) of the haemodialysis apparatus (10),
- Filling of the buffer volume (24) and building up of an admission pressure, until the pressure control valve opens,
- Closing of the first magnetic valve (22),
- Switching of the suction side of the degassing pump (36) from the piping system (34) to a feed pipe (46) for disinfecting fluid,
- Drawing off of disinfecting fluid,
- Switching of the suction side of the degassing pump (36) from the feed pipe (46) for disinfecting fluid to the piping system (34),
- Circulation of the disinfecting fluid and rinsing of the piping system (34) with a rinsing pressure Pₛ < Pₚᵥ,
- Monitoring of the signal delivered by the pressure sensor (P) and switching off of the pump (36) driving the disinfecting fluid as soon as the pressure of the buffer volume (24) falls below a predetermined limiting value.
- Opening of the first magnetic valve (22),
- Drawing off of the buffer volume (24),
- and drawing off of dialysis fluid from the supply pipeline (12).

## Revendications

1. Circuit hydraulique de sécurité en liaison avec un appareil d'hémodialyse (10), qui doit être raccordé au moyen d'un premier raccord (16) à une ligne d'alimentation (12), qui comporte, côté appareil, en amont du raccord (16), un premier organe de blocage (22) ainsi qu'un système ramifié de conduites tubulaires (34), qui se raccorde à cet organe de blocage et qui, par l'intermédiaire d'un second raccord (46), peut être balayé par un liquide de nettoyage et/ou un liquide désinfectant,
caractérisé par
un second organe de blocage (20), qui est disposé entre le premier raccord (16) et le premier organe de blocage (22) et qui réalise un blocage dans la direction allant de l'appareil d'hémodialyse (10) à la conduite d'alimentation (12), de sorte qu'est formé un volume tampon (24) disposé entre le second organe de blocage (20) et le premier organe de blocage (22),
des moyens (36) pour charger le volume tampon (24) avec une pression définie,
un capteur de pression (P) relié au volume tampon (24), et une unité d'évaluation pour un signal produit par le capteur de pression (P).

2. Circuit hydraulique de sécurité selon la revendication 1, caractérisé en ce que le premier organe de blocage (22) est une soupape magnétique (première soupape magnétique).

3. Circuit hydraulique de sécurité selon l'une des revendications 1 ou 2, caractérisé en ce que le second organe de blocage (20) est une soupape magnétique (seconde soupape magnétique).

4. Circuit hydraulique de sécurité selon l'une des revendications 1 ou 2, caractérisé en ce que le second organe de blocage (20) est une soupape antiretour et que la pression définie, qui est produite dans le volume tampon (24), est supérieure à la pression régnant dans la conduite d'alimentation (12) ainsi qu'à la pression de balayage qui règne, côté appareil, en amont du premier organe de blocage (22).

5. Circuit hydraulique de sécurité selon l'une des revendications précédentes, caractérisé en ce que le capteur de pression (P) est un capteur de pression analogique.

6. Circuit hydraulique de sécurité selon l'une des revendications 1 à 4, caractérisé en ce que le capteur de pression (P) est un interrupteur qui agit en fonction de la pression.

7. Circuit hydraulique de sécurité selon l'une des revendications précédentes, caractérisé en ce que les moyens (36) pour charger en pression le volume tampon (24) sont une pompe (36).

8. Circuit hydraulique de sécurité selon la revendication 7, caractérisé en ce que la pompe (36) est une pompe à membrane de l'appareil de dialyse.

9. Circuit de sécurité selon la revendication 7, caractérisé en ce qu'une pompe de dégazage (36), qui est disposée dans l'appareil de dialyse (10) et sert à entraîner le liquide de dialyse dans une section de dégazage (40) peut être activée en tant que pompe (36) pour la charge en pression du volume tampon (24).

10. Circuit de sécurité selon l'une des revendications 7 à 9, caractérisé en ce que le côté refoulement de la pompe (36) peut être relié directement ou indirectement, par l'intermédiaire d'une troisième soupape magnétique (52), au raccord, situé du côté de l'appareil, du premier organe de blocage (22) qui ferme le volume tampon (24).

11. Circuit de sécurité selon la revendication 10, caractérisé par une soupape de limitation de pression (62), qui est reliée au raccord, situé côté appareil, du premier organe de blocage (22) qui limite le volume tampon (24).

12. Circuit de sécurité selon la revendication 11, caractérisé en ce que la valeur de déclenchement de la soupape de limitation de pression (62) est égale à 1,2 bar.

13. Circuit de sécurité selon l'une des revendications 7 à 9, caractérisé en ce que le côté aspiration de la pompe (36) peut être relié directement ou indirectement par l'intermédiaire d'une quatrième soupape magnétique (56) au raccord, situé côté appareil, du premier organe de blocage (22), qui ferme le volume tampon (24).

14. Circuit de sécurité selon l'une des revendications 7 à 9, caractérisé en ce qu'un microprocesseur (28) est relié au moteur de la pompe (36).

15. Circuit de sécurité selon la revendication 14, caractérisé en ce que le microprocesseur (28) est relié électriquement à la troisième soupape magnétique (52).

16. Circuit de sécurité selon la revendication 14, caractérisé en ce que le microprocesseur (28) est relié électriquement à la quatrième soupape magnétique (56).

17. Circuit de sécurité selon la revendication 14, caractérisé en ce que le microprocesseur (28) est relié à la première soupape magnétique (22) et que la combinaison formée par le capteur de pression (P), la première soupape magnétique (22) et le microprocesseur (28) travaille en tant que réducteur de pression lorsque l'appareil d'hémodialyse (10) fonctionne selon le procédé régulier de dialyse et que la pression Pᵥ dans la conduite d'alimentation (12) du liquide de dialyse est supérieure à une valeur limite prédéterminée.

18. Procédé pour balayer le système de conduites tubulaires (34) d'un appareil d'hémodialyse (10) comportant un circuit de sécurité selon une ou plusieurs des revendications 10 à 17, comprenant un liquide de nettoyage et/ou désinfectant, lors du contrôle simultané du fonctionnement correct du premier organe de blocage (22) de la conduite d'alimentation (12), caractérisé par les étapes opératoires suivantes :
- ouverture de la première soupape magnétique (22),
- ouverture de la troisième soupape magnétique (52),
- aspiration du liquide d'osmose au moyen de la pompe de dégazage (36) à partir du système de conduites tubulaires (34) de l'appareil d'hémodialyse (10),
- remplissage du volume tampon (24) et établissement d'une pression préalable, jusqu'à ce que la soupape de régulation de pression s'ouvre,
- fermeture de la première soupape magnétique (22),
- commutation du côté aspiration de la pompe de dégazage (36) depuis le système de conduites tubulaires (34) à une canalisation d'arrivée (46) pour le fluide désinfectant,
- aspiration du liquide désinfectant,
- commutation du côté aspiration de la pompe de dégazage (36) depuis la canalisation d'amenée (46) pour le liquide désinfectant au système de conduites tubulaires (34),
- circulation du liquide désinfectant et balayage du système de conduites tubulaires (34) avec une pression de balayage P_{S} < P_{PV},
- contrôle du signal délivré par le capteur de pression (P) et débranchement de la pompe (36) entraînant le liquide désinfectant dès que la pression du volume tampon (24) tombe au-dessous d'une valeur limite prédéterminée,
- ouverture de la première soupape magnétique (22),
- aspiration du volume tampon (24), et
- aspiration du liquide de dialyse à partir de la conduite d'alimentation (12).
